(19) Europäisches Patentamt / European Patent Office / Office européen des brevets

(11) **EP 4 272 676 A1**

(12) **EUROPEAN PATENT APPLICATION**

(43) Date of publication:
**08.11.2023 Bulletin 2023/45**

(21) Application number: **23170657.3**

(22) Date of filing: **28.04.2023**

(51) International Patent Classification (IPC):
**A61B 18/14** *(2006.01)* **A61B 18/12** *(2006.01)*
**A61B 18/00** *(2006.01)*

(52) Cooperative Patent Classification (CPC):
**A61B 18/1492; A61B 18/1206;** A61B 2018/00613;
A61B 2018/00684; A61B 2018/00708;
A61B 2018/00761; A61B 2018/00988

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.05.2022 US 202263337209 P**
**20.03.2023 US 202318123424**

(71) Applicant: **BIOSENSE WEBSTER (ISRAEL) LTD.**
**2066717 Yokneam (IL)**

(72) Inventors:
• **GOVARI, Assaf**
**2066717 Yokneam (IL)**
• **ALTMANN, Andres Claudio**
**2066717 Yokneam (IL)**
• **MARZIANO, Lilah**
**2066717 Yokneam (IL)**

(74) Representative: **Carpmaels & Ransford LLP**
**One Southampton Row**
**London WC1B 5HA (GB)**

(54) **IRREVERSIBLE-ELECTROPORATION (IRE) WORKFLOW TO REDUCE TIME BETWEEN ABLATIONS**

(57) An irreversible electroporation (IRE) method includes receiving a total number of IRE pulses to be applied by one or more electrodes of a catheter placed in proximity to a tissue in an organ. An IRE protocol is defined by defining a partitioning of the total number of the IRE pulses into multiple pulse trains separated by pauses, the partitioning defined so as to reduce a total duration of the IRE protocol while meeting a safety criterion. The IRE protocol is applied to the tissue using the electrodes.

FIG. 2

**Description**

**CROSS-REFERENCE TO RELATED APPLICATION**

This application claims the benefit of U.S. Provisional Patent Application 63/337,209, filed May 2, 2022, which is incorporated herein by reference.

**FIELD OF THE DISCLOSURE**

The present disclosure relates generally to invasive ablation, and particularly to irreversible electroporation (IRE) of cardiac tissue.

**BACKGROUND OF THE DISCLOSURE**

Estimation of invasive ablation parameters and controlling the ablation according to the estimation has been previously proposed in the patent literature.

Irreversible electroporation (IRE), also called Pulsed Field Ablation (PFA), may be used as an invasive therapeutic modality to kill tissue cells by subjecting them to high-voltage pulses. It is known to use a train (or more than one train) of microsecond-duration high-voltage electrical pulses. Typically, more than one pulse train is applied with a defined pause between pulses.

Although IRE is not based on heating, there is still some local heating during the ablation process that may result in bubble formation in surrounding blood. Bubble formation may potentially lead to embolic risk.

International Patent Application Publication WO 2021/207385 describes devices, systems and methods for delivering pulsed electric field (PEF) energy to tissue through one or more energy delivery bodies, each having one or more electrodes. The PEF energy is generated from a waveform having a variety of features. Waveform delays, such as inter-pulse delays, inter-cycle delays, inter-phase delays, inter-packet delays, inter-bundle delays, may be utilized within a treatment to obtain a desired outcome. In particular, these delays may be specifically manipulated to obtain particular desired outcomes. For example, one, some or all of these delays may be manipulated to control various aspects of PEF therapy so as to mitigate any associated risks, such as gas formation, electrical discharge, cavity formation, muscle contraction, and temperature rise, to name a few. In some examples, the delays distribute the period over which PEF energy is delivered, resulting in marked changes and optimization to the treatment delivery outcomes.

The present disclosure will be more fully understood from the following detailed description of the examples thereof, taken together with the drawings in which:

**BRIEF DESCRIPTION OF THE DRAWINGS**

Fig. 1 is a schematic, pictorial illustration of a catheter-based irreversible electroporation (IRE) system, in accordance with an example of the present disclosure;

Fig. 2 is a schematic, pictorial illustration of a sequence of IRE pulses that is partitioned in two different ways into pulse trains with a respective number of pauses between the pulse trains;

Fig. 3 is a flow chart that schematically illustrates a method for applying a sequence of IRE pulses partitioned to avoid bubbles while reducing total ablation time, in accordance with an example of the present disclosure; and

Fig. 4 is a flow chart that schematically illustrates a method for applying a sequence of IRE pulses partitioned to avoid bubbles while reducing total ablation time, in accordance with another example of the present disclosure.

**DETAILED DESCRIPTION OF EXAMPLES**

OVERVIEW

To avoid bubbles in blood of a cavity of the body, an IRE workflow protocol may require a pause between repetitive ablations of the cavity wall tissue. As noted above, a typical pause may last about ten seconds. The pauses between repetitive ablations add significantly to the duration of the ablation procedure. The present inventors have surprisingly found that the duration of the pause needed to avoid bubble formation is highly sensitive to the pulse train length and/or the number of pulses included in a pulse train. A small reduction in pulse train length may significantly reduce the pause required to avoid bubble formation. The present inventors have further surprisingly found that the same number of pulses may be delivered to a patient over a shorter duration and without significant bubble formation based on dividing the number of pulses delivered into more pulse trains and reducing the pause duration between the pulse trains. According to some example embodiments, there is provided a method for reducing the duration of a IRE procedure while avoiding bubble formation related to IRE. If the duration of each of the repetitive epochs of IRE can be reduced, the risk of bubbles is reduced. At the same time, using shorter-duration pauses between epochs of ablation becomes possible, and the result is an overall faster and more consistent IRE procedure, to the benefit of the patient.

Examples of the present disclosure that are described hereinafter utilize an observation by the developers of the disclosed technique that the duration of the pause needed to avoid bubble formation may be significantly reduced by decreasing the length of the train of pulses in each IRE epoch, e.g., using fewer pulses per train. Based on empirical observations, the developers devised a method to reduce the overall duration of the pauses required to avoid bubble formation during an ablation procedure without reducing the overall energy applied to the tissue for ablation. By reducing the overall duration of the pauses, the duration of the ablation procedure may be significantly reduced.

To maintain the total number of pulses applied to tissue, the number of repetitions is increased so that, overall, the same number of pulses is delivered. Surprisingly, increasing the number of repetitions while reducing the duration of each train of pulses significantly decreases the pause length required to avoid bubble formation, which significantly decreases the overall ablation time required. For example, it was empirically found by the developers that three repetitions with a ten-second pause between them (resulting in a total pause of ~20 seconds) may be replaced by a sequence of four repetitions with a three-second pause between them (resulting in a total pause of ~9 seconds). Given that the pauses account for a significant portion of the total ablation time, the reduction in time duration of the ablation is significant.

In some examples, the disclosed techniques provide a lookup table that is stored in a memory of the IRE system. The lookup table gives an empirically derived relation between the total number of pulses to be applied, pulse voltage, and pulse repetition rate, arriving at an optimal number, and duration of pauses that, if chosen, reduces total time duration of ablation, and, on the other hand, also meets safety criterion (e.g., not to cause bubbles).

In another example, a processor uses a set of electrode heat generation rate and blood heat removal rate equations to calculate an optimal number and duration of pulse trains (e.g., repetitive epochs of ablation) and duration of respective pauses. The equations take into account total electrical power applied in each epoch of the pulses, of which a fraction is dissipated, and the heat removal rate in blood. Solving the equations can be subjected to a constraint on the maximal amount of heat generation allowed per each IRE epoch to find the maximal epoch duration allowed, and using the maximal amount of heat generated also to calculate a reduced duration of a subsequent pause.

Further reduction of pulse train duration and corresponding reduction of the duration for blood cooling (e.g., duration of pauses) will require more repetitions. A possible target of the optimization may be to achieve a reduced total time of an IRE session which is a weighted sum of the durations (weighted by heat generation and heat dissipation rates) over the number of repetitions.

In one example, a user selects a total number of IRE pulses to be applied by one or more electrodes of a catheter placed in contact with tissue in an organ. The user defines an IRE protocol by defining a partitioning of the total number of the IRE pulses into multiple pulse trains separated by pauses, the partitioning defined so as to reduce a total duration of the IRE treatment while meeting the safety criterion (e.g., that no bubbles are formed, or a measured temperature does not exceed a given value). The user applies the IRE protocol to the tissue using the electrodes. Defining the partitioning comprises choosing a number of the pauses and respective durations of the pauses that reduces the total duration while meeting the safety criterion.

In yet another example, the processor optimizes the pauses between individual pulses in a train of pulses, as well as the pauses between the trains, both for the purpose of reducing the overall time required to complete all the ablations while avoiding bubble formation. The parameters that the processor may select to reduce the overall duration are pauses between trains, pauses between pulses, and number of pulses per train.

SYSTEM DESCRIPTION

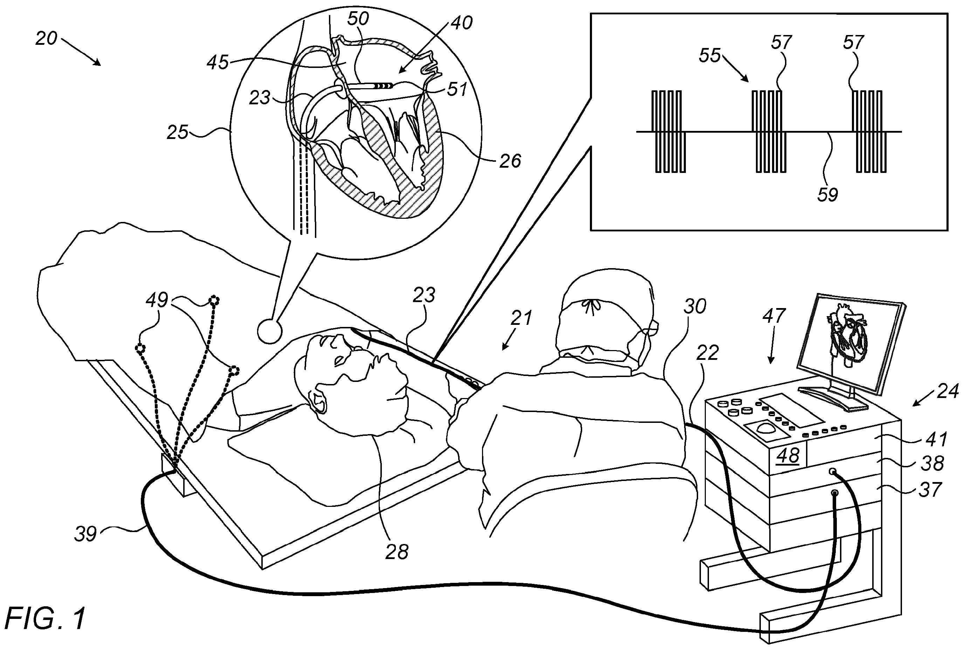

Fig. 1 is a schematic, pictorial illustration of a catheter-based irreversible electroporation (IRE) system 20, in accordance with an example of the present disclosure. System 20 comprises a catheter 21, wherein a shaft 22 of the catheter is inserted by a physician 30 through the vascular system of a patient 28 through a sheath 23. The physician then navigates a distal end 40 of shaft 22 to contact a target tissue location (e.g., a wall tissue of left atrium 45) inside a heart 26 of the patient.

As seen in an inset 25, the exemplified catheter 21 has a distal end 40 comprising ring electrodes 50 and a tip electrode 51. The electrodes can be used for bipolar and/or unipolar IRE. The proximal end of catheter 21 is connected to a console 24 comprising an IRE pulse generator 38 configured to apply the IRE pulses between adjacent pairs of electrodes among electrodes 50 and 51 in a bipolar IRE mode, or between electrode 51 and a body surface electrode 49 in a unipolar IRE mode. The electrodes are connected to IRE pulse generator 38 by electrical wiring running in shaft 22 of catheter 21.

In the pictured example, after positioning distal end 50 at an IRE tissue site, and ensuring that the tip is in contact with the tissue, physician 30 actuates an IRE pulse generator 38 in a control console 24 to supply IRE pulses to electrodes 50 and/or 51 via a cable 38. Optionally, contact is not required for activating IRE ablation.

A memory 48 of console 24 stores predefined IRE protocols comprising IRE parameters, such as those shown in Table I below.

Console 24 comprises a processor 41, typically a general-purpose computer, with suitable front end and interface circuits 37 for, optionally, receiving signals from catheter 21 and from external electrodes 49, which are typically placed around the chest of patient 26. For this purpose, processor 41 is connected to external electrodes 49 by wires running through a cable 39.

During a procedure, optionally, system 20 can track the respective locations of electrodes 50 inside heart 26 using the Active Current Location (ACL) method, which is described in US Patent 8,456,182, whose disclosure is incorporated herein by reference.

In some examples, the physician provides input on where he wants to ablate. Depending on the parameters of that location (such as wall thickness and tissue type), the protocol may be determined by processor 41, including the processor dividing (partitioning) the IRE pulse delivery 55 of the selected protocol into multiple pulse trains 57 with pauses 59 between pulse trains. The pauses permit Joule heating from any pulse to dissipate sufficiently to prevent bubble formation.

In case physician 30 is worried of a risk of bubbles using an initially defined (e.g., preset) IRE protocol, physician 30 may instruct processor 41 to use a more cautious protocol: As a first example, the physician can modify, via a user interface 47, the length of each train and the number of repetitions. Based on the selection, the program will define the length of the pause required. As a secondary option, the user can define two out of the three parameters (train length, repetitions and pause length), and the third parameter will be computed. As a third option, the user can define any or all of the three parameters, and the algorithm will provide warnings when the protocol can lead to bubbles.

User interface 47 may comprise any suitable type of input device, e.g., keyboard, mouse, trackball and other similar devices.

Processor 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

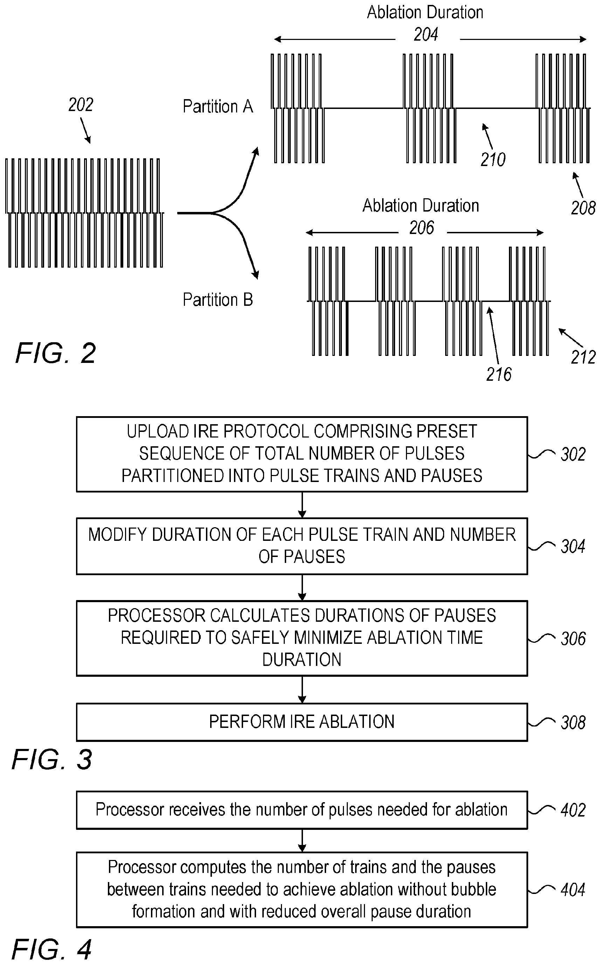

In particular, processor 41 runs a dedicated algorithm as disclosed herein and shown in Fig. 3, which enables processor 41 to perform the disclosed steps, as further described below. In particular, processor 41 is configured to command IRE pulse generator 38 to output IRE pulses according to a treatment protocol that processor 41 uploads from memory 48.

IRE WORKFLOW FOR REDUCED ABLATION TIME WHILE AVOIDING BUBBLE GENERATION

Fig. 2 is a schematic, pictorial illustration of a sequence 202 of IRE pulses that is partitioned in two different ways, A and B, into pulse trains with a respective number of pauses between the pulse trains. As seen in partition A, sequence 202 is divided into three pulse trains 208 with two pauses 210 in between, which sum to a total time duration 204 of ablation. In partition B, sequence 202 is divided into four shorter pulse trains 212 with three considerably shorter pauses 216 between them, which sum to a shorter total time duration 206 of ablation.

As an example, a sequence is considered, such as sequence 202 comprising 24 bipolar pulses with a repetition rate of 4 Hz, the sequence lasting six seconds. Partition A divides the sequence into three pulse trains of eight pulses (each train thus lasting two seconds) with two three-second pauses between them. The total ablation time in partition A is therefore 12 seconds.

Partition B divides the sequence into four pulse trains of six pulses, each train lasting 1.5 seconds, with three one-second pauses between sets. The total ablation time in partition B is therefore reduced to 7.5 seconds, a 45% reduction in time duration of the ablation.

In one example, the disclosed technique provides a lookup table that is stored in a memory of the IRE system. The lookup table takes an empirically derived relation between the total number of pulses to be applied, pulse voltage, and pulse repetition rate, to provide an optimal number and duration of pauses that, on the one hand, reduces the total time duration of ablation, and, on the other hand, meets a safety criterion, such as not causing bubbles. Table I below is an example of a portion of such a possible lookup table.

Table I

| Total # pulses (N) | Voltage [V] | Number of pulses per train (N/(k+1)) | Repetition rate [Hz] f | Pause [Sec] | # Pauses (k) | Total duration [Sec] |
|---|---|---|---|---|---|---|
| 40 | 1000 | 10 | 4 | 3 | 3 | 19 |

(continued)

| Total # pulses (N) | Voltage [V] | Number of pulses per train (N/(k+1)) | Repetition rate [Hz] f | Pause [Sec] | # Pauses (k) | Total duration [Sec] |
|---|---|---|---|---|---|---|
| 40 | 2000 | 5 | 4 | 2 | 7 | 24 |
| 40 | 1000 | 5 | 8 | 1 | 7 | 12 |
| 24 | 1000 | 6 | 4 | 2 | 3 | 12 |

In another example, a processor uses a set of equations, similar to Eq. 1 below, to calculate the optimal number and duration of the IRE pulse trains and the pauses in between. The equation takes into account the maximal allowed blood temperature, total electrical power applied in each epoch, of which a fraction is deposited in blood, and blood heat removal capacity to calculate a reduced but still safe cooling pause duration.

In an example, a total time can be solved as a sum of heating and cooling periods using, for example, heat generation and dissipation rate equations:

$$\text{Eqs. 1} \qquad \begin{cases} \dfrac{k+1}{N} \propto V^2 \\ \log\left(\dfrac{k+1}{N/f}\right) \propto -\dfrac{h \cdot N}{k \cdot f} \end{cases}$$

In Eq. 1, k is the number of pauses, $N/f$ is the total duration for applying a number N of pulses in an uninterrupted sequence at a reputation rate f of the pulses, V is the pulse voltage, and h is the rate of heat removal from the location of an electrode, which is governed by blood flow.

Fig. 3 is a flow chart that schematically illustrates a method for applying sequence 202 of IRE pulses, partitioned to avoid bubbles, in accordance with an example of the present disclosure. By reducing the duration of each ablation pulse train in a sequence of ablation pulse trains, the disclosed technique is able to provide shorter ablation periods. Moreover, by introducing more pauses that are each shorter, the technique achieves a reduced total ablation time, as illustrated, for example, in Fig. 2 by new duration 206 versus preset duration 204.

The algorithm, which can be run by system 20, according to the presented example, carries out a process that begins when physician 30 uploads an IRE protocol comprising a preset sequence of a total number of pulses partitioned into pulse train and pauses, such as in partition A of Fig. 2, at an IRE protocol uploading step 302.

Next, at an IRE protocol modification step 304, as an example, the physician modifies, from a user interface 47, the length of each train 208 (e.g., into train 212) and the number of repetitions (e.g., from three to four as partition B of Fig. 2 shows).

Then, at an adjustment step 306, based, for example, on lookup table I, processor 41 calculates the new durations (e.g., duration 216) of pauses, for example, using Table I, so as to safely reduce the total ablation time duration (e.g., from duration 204 to duration 206).

Finally, processor 41 commands generator 38 to apply the partitioned IRE pulses to tissue, at an IRE treatment step 308. The IRE pulses are applied for example, bipolarly between selected electrodes among electrodes 50 and 51, to isolate an arrhythmia in left atrium 45.

Fig. 4 is a flow chart that schematically illustrates a method for applying a sequence 202 of IRE pulses partitioned to avoid bubbles while reducing total ablation time, in accordance with another example of the present disclosure. The algorithm, which can be run by system 20, according to the presented example, carries out a process that begins when processor 41 receives the number of pulses needed for ablation, at IRE parameters receiving step 402.

Next, at an IRE protocol generation step 404, processor 41 computes the number of trains and the pauses between the trains required to achieve the ablation needed without bubble formation and with pauses of reduced duration (or to achieve reduced overall pause duration for the entire procedure).

EXAMPLES

Example 1

An irreversible electroporation (IRE) method includes receiving a total number of IRE pulses to be applied by one or more electrodes (50, 51) of a catheter (21) placed in proximity to a tissue in an organ. An IRE protocol is defined by defining a partitioning of the total number of the IRE pulses into multiple pulse trains (212) separated by pauses (216), the partitioning defined so as to reduce a total duration (206) of the IRE protocol while meeting a safety criterion. The IRE protocol is applied to the tissue using the electrodes.

Example 2

The method according to example 1, wherein defining the partitioning comprises choosing a number of the pauses and respective durations of the pauses, that reduces the total duration while meeting the safety criterion.

Example 3

The method according to examples 1 or 2, wherein choosing a number of the pauses and respective durations of the pauses comprises using a look-up table.

Example 4

The method according to any of examples 1 through 3, wherein the look-up table determines the number of pauses based on the total number of IRE pulses, pulse voltage, and pulse repetition rate.

Example 5

The method according to example 2, wherein choosing a number of the pauses and respective durations of the pauses comprises using a set of equations.

Example 6

An irreversible electroporation (IRE) system 20 includes a user interface (47) and a processor (41). The user interface is configured for selecting a total number of IRE pulses to be applied by one or more electrodes of a catheter placed in proximity with tissue in an organ. The processor is configured to (i) define an IRE protocol by defining a partitioning of the total number of the IRE pulses into multiple pulse trains separated by pauses, the partitioning defined so as to reduce a total duration of the IRE protocol while meeting a safety criterion, and (ii) apply the IRE protocol to the tissue using the electrodes.

Example 7

An irreversible electroporation (IRE) system 20 includes a memory (48) and a processor (41). The memory (48) is configured to store an IRE protocol comprising a total number of IRE pulses to be applied by one or more electrodes of a catheter placed in proximity with tissue in an organ. The processor (41) is configured to (i) upload the IRE protocol from the memory, (ii) modify an IRE protocol by defining a partitioning of the total number of the IRE pulses into multiple pulse trains separated by pauses, the partitioning defined so as to reduce a total duration of the IRE protocol while meeting a safety criterion, and (iii) apply the IRE protocol to the tissue using the electrodes.

Although the examples described herein mainly address cardiac applications, the methods and systems described herein can also be used in other medical applications, such as in neurology and otolaryngology.

It will thus be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

**Claims**

1.   An irreversible electroporation (IRE) system, comprising:

a user interface configured for receiving a total number of IRE pulses to be applied by one or more electrodes of a catheter placed in proximity with tissue in an organ; and
a processor, which is configured to:

define an IRE protocol by defining a partitioning of the total number of the IRE pulses into multiple pulse trains separated by pauses, the partitioning defined so as to reduce a total duration of the IRE protocol while meeting a safety criterion; and
apply the IRE protocol to the tissue using the electrodes.

2. The system according to claim 1, wherein the processor is configured to define the partitioning by choosing a number of the pauses and respective durations of the pauses, that reduces the total duration while meeting the safety criterion.

3. The system according to claim 2, wherein the processor is configured to choose a number of the pauses and respective durations of the pauses by using a look-up table.

4. The system according to claim 3, wherein the look-up table determines the number of pauses based on the total number of IRE pulses, pulse voltage, and pulse repetition rate.

5. The system according to claim 2, wherein the processor is configured to choose a number of the pauses and respective durations of the pauses by using a set of equations.

6. The system according to claim 1, comprising:

a memory configured to store an IRE protocol comprising a total number of IRE pulses to be applied by one or more electrodes of a catheter placed in proximity with tissue in an organ; and
wherein the processor is configured to:

upload the IRE protocol from the memory; and
modify the IRE protocol by defining a partitioning of the total number of the IRE pulses into multiple pulse trains separated by pauses, the partitioning defined so as to reduce a total duration of the IRE protocol while meeting a safety criterion.

FIG. 1

Ablation Duration
204

Partition A

202

210

208

Ablation Duration
206

Partition B

212

216

**FIG. 2**

| UPLOAD IRE PROTOCOL COMPRISING PRESET SEQUENCE OF TOTAL NUMBER OF PULSES PARTITIONED INTO PULSE TRAINS AND PAUSES | 302 |

↓

| MODIFY DURATION OF EACH PULSE TRAIN AND NUMBER OF PAUSES | 304 |

↓

| PROCESSOR CALCULATES DURATIONS OF PAUSES REQUIRED TO SAFELY MINIMIZE ABLATION TIME DURATION | 306 |

↓

| PERFORM IRE ABLATION | 308 |

**FIG. 3**

| Processor receives the number of pulses needed for ablation | 402 |

↓

| Processor computes the number of trains and the pauses between trains needed to achieve ablation without bubble formation and with reduced overall pause duration | 404 |

**FIG. 4**

Europäisches
Patentamt

European
Patent Office

Office européen
des brevets

# EUROPEAN SEARCH REPORT

**Application Number**

EP 23 17 0657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2022/031387 A1 (GOVARI ASSAF [IL]) 3 February 2022 (2022-02-03) * paragraph [0005] - paragraph [0017] * * paragraph [0029] - paragraph [0037] * * figures 1-2 * | 1-5 | INV. A61B18/14 A61B18/12 ADD. A61B18/00 |
| Y | US 2021/401490 A1 (GOVARI ASSAF [IL] ET AL) 30 December 2021 (2021-12-30) * paragraph [0029] - paragraph [0034] * * paragraph [0056] - paragraph [0068] * * figure 6 * | 1-5 | |
| Y | US 2022/071692 A1 (GOVARI ASSAF [IL] ET AL) 10 March 2022 (2022-03-10) * paragraph [0004] - paragraph [0017] * * paragraphs [0032], [0038]; claim 2 * | 3-5 | |
| A | WO 2021/207385 A1 (GALARY INC [US]; CASTELLVI QUIM [ES] ET AL.) 14 October 2021 (2021-10-14) * paragraph [0255] - paragraph [0291]; figures 11A-D, 21A-H * | 1-6 | TECHNICAL FIELDS SEARCHED (IPC) A61B |
| A | US 2019/201089 A1 (WALDSTREICHER JONATHAN REUBEN [US] ET AL) 4 July 2019 (2019-07-04) * paragraph [0099] - paragraph [0116] * * paragraph [0261] - paragraph [0263] * | 1-6 | |

The present search report has been drawn up for all claims

| Place of search | Date of completion of the search | Examiner |
|---|---|---|
| The Hague | 15 August 2023 | Hilvert, Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

**ANNEX TO THE EUROPEAN SEARCH REPORT**
**ON EUROPEAN PATENT APPLICATION NO.** EP 23 17 0657

This annex lists the patent family members relating to the patent documents cited in the above-mentioned European search report.
The members are as contained in the European Patent Office EDP file on
The European Patent Office is in no way liable for these particulars which are merely given for the purpose of information.

15-08-2023

| Patent document cited in search report | | | Publication date | Patent family member(s) | | | Publication date |
|---|---|---|---|---|---|---|---|
| US 2022031387 | A1 | | 03-02-2022 | CN | 113995498 | A | 01-02-2022 |
| | | | | EP | 3944829 | A1 | 02-02-2022 |
| | | | | IL | 281790 | A | 01-02-2022 |
| | | | | JP | 2022024985 | A | 09-02-2022 |
| | | | | KR | 20220014274 | A | 04-02-2022 |
| | | | | US | 2022031387 | A1 | 03-02-2022 |
| US 2021401490 | A1 | | 30-12-2021 | CN | 113925596 | A | 14-01-2022 |
| | | | | EP | 3932349 | A1 | 05-01-2022 |
| | | | | IL | 281801 | A | 01-01-2022 |
| | | | | JP | 2022013664 | A | 18-01-2022 |
| | | | | KR | 20220001439 | A | 05-01-2022 |
| | | | | US | 2021401490 | A1 | 30-12-2021 |
| US 2022071692 | A1 | | 10-03-2022 | CN | 114145837 | A | 08-03-2022 |
| | | | | EP | 3964153 | A1 | 09-03-2022 |
| | | | | IL | 281795 | A | 01-04-2022 |
| | | | | JP | 2022045316 | A | 18-03-2022 |
| | | | | KR | 20220033002 | A | 15-03-2022 |
| | | | | RU | 2770452 | C1 | 18-04-2022 |
| | | | | US | 2022071692 | A1 | 10-03-2022 |
| WO 2021207385 | A1 | | 14-10-2021 | AU | 2021251864 | A1 | 03-11-2022 |
| | | | | CA | 3179790 | A1 | 14-10-2021 |
| | | | | CN | 115348844 | A | 15-11-2022 |
| | | | | EP | 4132400 | A1 | 15-02-2023 |
| | | | | JP | 2023521745 | A | 25-05-2023 |
| | | | | US | 2023172650 | A1 | 08-06-2023 |
| | | | | WO | 2021207385 | A1 | 14-10-2021 |
| US 2019201089 | A1 | | 04-07-2019 | AU | 2017289267 | A1 | 17-01-2019 |
| | | | | AU | 2021266354 | A1 | 09-12-2021 |
| | | | | CA | 3029260 | A1 | 04-01-2018 |
| | | | | CN | 109788979 | A | 21-05-2019 |
| | | | | CN | 114098949 | A | 01-03-2022 |
| | | | | DK | 3474760 | T3 | 20-03-2023 |
| | | | | EP | 3474760 | A1 | 01-05-2019 |
| | | | | EP | 4209190 | A1 | 12-07-2023 |
| | | | | JP | 7287888 | B2 | 06-06-2023 |
| | | | | JP | 2019524396 | A | 05-09-2019 |
| | | | | JP | 2022109958 | A | 28-07-2022 |
| | | | | US | 2019201089 | A1 | 04-07-2019 |
| | | | | US | 2019231425 | A1 | 01-08-2019 |
| | | | | US | 2020323586 | A1 | 15-10-2020 |
| | | | | US | 2022395323 | A1 | 15-12-2022 |
| | | | | WO | 2018005511 | A1 | 04-01-2018 |

For more details about this annex : see Official Journal of the European Patent Office, No. 12/82

EPO FORM P0459

## ANNEX TO THE EUROPEAN SEARCH REPORT
## ON EUROPEAN PATENT APPLICATION NO.

EP 23 17 0657

This annex lists the patent family members relating to the patent documents cited in the above-mentioned European search report.
The members are as contained in the European Patent Office EDP file on
The European Patent Office is in no way liable for these particulars which are merely given for the purpose of information.

15-08-2023

| Patent document cited in search report | Publication date | Patent family member(s) | Publication date |
| --- | --- | --- | --- |

For more details about this annex : see Official Journal of the European Patent Office, No. 12/82

**REFERENCES CITED IN THE DESCRIPTION**

**Patent documents cited in the description**

- US 63337209
- WO 2021207385 A
- US 8456182 B